# FASCICULE DE BREVET EUROPEEN

(11) **EP 3 386 404 B1**
(45) Date de publication et mention de la délivrance du brevet: **11.03.2020**
(21) Numéro de dépôt: 16810331.5
(22) Date de dépôt: 09.12.2016
(51) Int. Cl.: A61B 17/66, A61B 17/68

(54) **ENSEMBLE D'UN DISTRACTEUR A PLAQUES ET D'UN OUTIL D ACTIVATION**
ANORDNUNG EINES PLATTENDISTRAKTORS UND EINES AKTIVIERUNGSINSTRUMENTS
ASSEMBLY OF A PLATE DISTRACTOR AND AN ACTIVATION TOOL

(30) Priorité: 09.12.2015 FR 1562069
(43) Date de publication de la demande: 17.10.2018
(73) Titulaire: École Nationale Supérieure de Techniques Avancées Bretagne, 91120 Palaiseau (FR); Ecole Polytechnique, 91128 Palaiseau Cedex (FR); ASSISTANCE PUBLIQUE - HÔPITAUX DE PARIS, 75004 Paris (FR); Université Paris Descartes, 75270 Paris Cedex 06 (FR)
(72) Inventeur: STROZYK, Hervé, 57490 L'hôpital (FR); BOISSON, Jean, 75020 Paris (FR); CHERFA, Lahcen, 91220 Brétigny-sur-Orge (FR); KADLUB, Natacha, 75015 Paris (FR); PICARD, Arnaud, 75015 Paris (FR)
(74) Mandataire: LLR
(86) Numéro de dépôt international: PCT/EP2016/080481
(87) Numéro de publication internationale: WO 2017/097998

(56) Documents cités:
- WO-A1-01/78614
- WO-A1-99/51160
- WO-A1-2008/003952
- WO-A2-2007/144489
- US-A1- 2008 108 995

## Description

La présente invention se rapporte à un distracteur à plaques, notamment à un distracteur maxillo-facial à plaques. L'invention vise également un ensemble d'un tel distracteur et d'un outil d'activation du distracteur.

Dans certaines situations cliniques, le retard ou l'absence de croissance mandibulaire impose une chirurgie d'allongement de la mandibule. Afin de ne pas interférer ou bloquer la croissance naturelle, il est réalisé une distraction ostéogénique. Cette technique chirurgicale permet un allongement progressif des os. Pour ce faire, on réalise tout d'abord une section ou fracture chirurgicale de l'os, appelée ostéotomie. Ensuite, on fixe deux plaques d'un distracteur à plaques sur l'os, de part et d'autre de l'ostéotomie. Un dispositif de vis sans fin permet de contrôler l'écart entre les plaques et, ainsi, de provoquer l'allongement de l'os mandibulaire.

Il est connu de coupler le dispositif à vis sans fin à une tige articulée pour commander l'écartement des plaques, par rotation de ladite tige, de manière plus aisée. Toutefois, pendant toute la période de traitement, le distracteur avec la tige articulée reste dans la bouche, ce qui peut gêner l'alimentation, blesser les muqueuses et/ou être un obstacle à la fermeture de la bouche.

De plus, la tige articulée traverse la peau ou la muqueuse pour pouvoir être manipulée. Cette solution engendre donc des risques de complications infectieuses, notamment car elle met en contact l'os avec l'environnement extérieur.

Il est connu de WO 99/51160 un distracteur à plaques dont l'écartement des plaques est contrôlé par un système magnétique.

Il est connu de la demande internationale WO 2008/003952 un distracteur à plaques comportant deux plaques dont l'écartement est commandé à l'aide d'un aimant permanent relié aux plaques par une vis et entraîné en rotation par un actionneur magnétique rotatif disposé latéralement par rapport à l'aimant permanent. Les deux plaques sont maintenues fixes en rotation par deux tiges parallèles le long desquelles elles peuvent coulisser. Une telle disposition de l'actionneur magnétique par rapport à l'aimant permanent exerce une force transversale sur les plaques par l'intermédiaire de l'aimant permanent, ce qui peut entraîner un arrachement des plaques des os sur lesquelles elles sont fixées. En effet, un distracteur magnétique est conçu pour résister à des forces longitudinales mais présente une résistance plus faible aux forces transversales. De plus, dans un tel dispositif, les éléments rotatifs du distracteur, en particulier, l'aimant permanent et la vis ne sont pas protégés de leur environnement. Il peut donc y avoir des adhérences des tissus qui peuvent s'arracher à l'actionnement du distracteur à plaques.

L'invention vise donc à proposer un distracteur à plaques, notamment un distracteur maxillo-facial à plaques, ne présentant pas les inconvénients susmentionnés.

Il est à noter qu'il existe, outre les distracteurs à plaques se fixant sur les os à traiter, des distracteurs dits centromédullaires, qui sont insérés dans l'os à traiter.

Il est connu de commander l'allongement du distracteur centromédullaire par coopération d'un aimant intégré dans le distracteur centromédullaire et d'un aimant à l'extérieur du patient, la rotation de l'aimant dans le distracteur centromédullaire commandant la rotation du dispositif à vis sans fin.

La demande WO-A-0178614 décrit un exemple d'un tel distracteur centromédullaire. Dans ce document, un électro-aimant est disposé tout autour de l'os à traiter. Cet électro-aimant est commandé pour créer un champ magnétique variable provoquant la rotation de l'aimant inséré dans le distracteur centromédullaire. Une telle solution est toutefois difficile à mettre en œuvre pour un distracteur maxillo-faciale, car l'électro-aimant devrait faire le tour de la tête du patient. Plus généralement, cette solution n'est pas opportune pour un distracteur à plaques, fixé sur un côté d'un os. En effet, dans ce cas, l'action du champ magnétique sur l'aimant dans le distracteur serait variable en fonction de l'angle du champ magnétique. Ceci provoquerait des variations de couple sur un tour de rotation du système à vis sans fin. La commande de l'allongement d'un tel distracteur serait donc complexe. De plus, dans cette configuration la force d'interaction entre les aimants est perpendiculaire au plan des plaques. Cette force d'interaction exerce alors sur les vis, qui fixent les plaques à l'os, une contrainte dans la direction perpendiculaire au plan des plaques. Ceci augmente le risque d'arracher les plaques de l'os sur lequel elles sont fixées.

L'invention propose un ensemble de distraction tel que défini dans la revendication 1.

Dans l'invention, la rotation de la tige filetée par rapport au tube taraudé entraîne un déplacement de ce dernier le long de la tige filetée, ce qui permet l'allongement de la distance entre la deuxième plaque et la première plaque.

Ainsi, avantageusement, l'allongement du distracteur à plaques peut être commandé depuis l'extérieur du patient, sans contact et sans activateur traversant la peau ou la muqueuse. Il n'est plus nécessaire de mettre en œuvre une tige articulée traversant les tissus du patient. Le distracteur à plaques selon l'invention provoque ainsi moins de gênes.

En outre, il est possible de commander l'allongement du distracteur à plaques selon l'invention à l'aide d'un outil d'activation comprenant par exemple un aimant cylindrique magnétisé selon une direction transverse à l'axe de cet aimant cylindrique de l'outil d'activation. Il suffit dans ce cas de placer les deux aimants en vis-à-vis, la rotation de l'aimant de l'outil d'activation, autour de son axe, provoquant la rotation de l'aimant dans le distracteur à plaques. Il est à noter ici que cette rotation de l'aimant dans le distracteur est obtenue sans générer de force d'arrachement sur les vis fixant chaque plaque, perpendiculairement à la plaque.

Il est particulièrement intéressant que l'aimant soit situé à proximité d'une extrémité du distracteur à plaques, pour favoriser l'interaction entre l'aimant dans le distracteur à plaques et l'aimant dans l'outil d'activation.

La présence de la douille permet de protéger le tube taraudé de l'environnement extérieur et la tige filetée, ce qui améliore la sécurité du distracteur à plaques lors de son utilisation sur le patient.

La présence du logement hermétiquement clos permet d'isoler l'aimant du corps humain et d'éviter, lors de la fabrication du distracteur à plaques, que l'aimant se désaimante sous l'action des fortes températures de soudage des éléments entre eux.

En outre, du fait des directions de magnétisation des deux aimants, un effort d'attraction ou de répulsion entre ces deux aimants est réduits, par rapport à une configuration où les aimants interagissent selon la direction de leurs axes d'aimantation. On réduit ainsi les contraintes engendrées par le distracteur à plaque sur l'os, sur lequel le distracteur à plaques est fixé, ces contraintes étant susceptibles de provoquer l'arrachement du distracteur à plaques.

Selon des modes de réalisation préférés, le distracteur à plaques selon l'invention présente une ou plusieurs des caractéristiques suivantes, prises seules ou en combinaison :
- l'aimant est situé à une extrémité du distracteur à plaques ;
- l'aimant est de forme générale cylindrique, notamment à base circulaire ou polygonale, de préférence à base hexagonale, ou sphérique ;
- le distracteur à plaques comprend un dispositif de renvoi d'angle de telle sorte que l'axe de rotation de l'aimant soit sensiblement perpendiculaire à l'axe de la tige filetée et du tube taraudé ;
- le dispositif de renvoi d'angle comporte une première roue dentée, solidaire en rotation de l'aimant et adaptée à engrener une deuxième roue dentée commandant la rotation de l'un parmi la tige filetée et le tube taraudé ;
- l'aimant est relié à la tige filetée ou au tube taraudé par un dispositif de réduction, notamment à engrenage ;
- le dispositif de réduction inclut, de préférence est constitué par, les première et deuxième roues dentées ;
- le distracteur à plaques comprend un bras relié au tube fileté ou, de préférence, à la tige filetée, par une liaison rotule à doigt et le dispositif de réduction de couple, le cas échéant, l'aimant étant fixé sur le bras ;
- la liaison rotule à doigt est réalisée au moyen d'un joint de cardan ;
- l'aimant est solidaire en rotation de la tige filetée ou du tube taraudé, l'aimant étant de préférence fixé à la tige filetée ou au tube taraudé ;
- le distracteur à plaques comprend un dispositif anti-retour empêchant la rotation de l'aimant dans un sens commandant une réduction de la distance entre les première et deuxième plaques, le dispositif anti-retour étant de préférence du type à cliquet ;
- l'aimant est en néodyme ou en un alliage de néodyme, en samarium ou alliage de samarium, en ferrite ou alliage de ferrite, de préférence enrobé d'un matériau biocompatible ;
- au moins l'un parmi, de préférence chacun parmi la tige filetée, le tube taraudé, la première plaque et la deuxième plaque est en titane ou en alliage de titane ;
- la première plaque est la plaque la plus proche de l'aimant ;
- la première plaque est la plaque la plus éloignée de l'aimant ;
- le logement est fixé à la tige filetée ;
- le logement est libre en rotation par rapport à la douille ;
- l'aimant est fixe dans le logement ;
- l'aimant et le logement sont configuré pour que l'aimant soit libre en rotation dans le logement lorsqu'il tourne dans un premier sens et que l'aimant entraîne en rotation le logement et de ce fait la tige filetée lorsqu'il tourne en rotation dans un second sens, notamment l'aimant comporte une encoche asymétrique et le logement comporte un relief saillant coopérant avec l'encoche de sorte que l'aimant soit libre de tourner dans le premier sens et que le relief vienne en prise avec l'encoche dans le second sens.
- la douille comporte une ouverture longitudinale de largeur sensiblement égale à la largeur de la jonction entre le tube taraudé et la deuxième plaque pour guider le tube taraudé en translation ;
- le tube taraudé présente une largeur inférieure ou égale à la largeur de la deuxième plaque ; une telle extension réduite du tube taraudé permet de limiter les frottements, notamment avec la douille lors de son déplacement ;
- l'aimant permanent est tel que son aimantation est conservée lorsque l'aimant permanent est chauffé à une température de 120°C, mieux de 130°C, encore mieux de 134°C ; ceci permet de stériliser le distracteur magnétique tout en conservant l'aimantation ;
- la tige filetée est montée libre en rotation et solidaire en translation par rapport à la première plaque de fixation, en particulier la première plaque est fixe par rapport à la douille ;
- la tige filetée comporte deux zones longitudinales ayant des filetages inversés, le premier tube taraudé sur lequel est fixé la deuxième plaque étant monté sur une des deux zones longitudinales et un deuxième tube taraudé sur lequel est fixé la première plaque étant monté sur l'autre des deux zones longitudinales. Ainsi, la rotation de la tige filetée entraîne une translation des deux tubes taraudés dans des directions opposées, ce qui permet d'écarter les plaques l'une par rapport à l'autre ;
- les spires du filet de la première zone longitudinale présentent un espacement identique ou différent des spires du filet de la deuxième zone longitudinale. En contrôlant les espacements entre les spires des deux zones longitudinales il est alors possible d'avoir un distracteur permettant un déplacement des plaques adapté à la morphologie du patient et à la zone à traiter ;
- le distracteur à plaques comprend en outre un dispositif de détermination de la longueur d'allongement du distracteur, notamment à cliquet.

L'invention se rapporte à un ensemble de distraction comprenant un distracteur à plaques tel que décrit ci-avant, dans toutes ses combinaisons, et un outil d'activation du distracteur à plaques comprenant un aimant permanent, s'étendant selon une direction principale et aimanté selon une direction transversale à la direction principale, destiné à être placé en regard de l'aimant du distracteur à plaques.

Ainsi, la rotation de l'aimant de l'outil d'activation autour de sa direction principale entraîne la rotation de l'aimant du distracteur à plaques autour de son axe.

Selon des modes de réalisation préférés, l'ensemble de distraction selon l'invention présente une ou plusieurs des caractéristiques suivantes, prises seules ou en combinaison :
- l'aimant de l'outil d'activation est cylindrique, notamment à base circulaire ou polygonale, de préférence à base hexagonale, ou sphérique ;
- l'aimant de l'outil d'activation s'étend selon l'axe longitudinal du distracteur à plaques. Ceci limite les risques d'arrachement du distracteur à plaque ;
- l'outil d'activation comporte un logement d'écrantage magnétique dans lequel l'aimant est reçu pouvant passer d'une configuration fermée dans laquelle l'écrantage du champ magnétique est suffisante pour que la rotation de l'aimant de l'outil d'activation autour de la direction principale n'entraîne pas de rotation de l'aimant du distracteur à plaque autour de son axe à une configuration ouverte dans laquelle la rotation de l'aimant de l'outil d'activation autour de la direction principale entraîne la rotation de l'aimant du distracteur à plaque autour de son axe et inversement. Un tel logement d'écrantage magnétique permet d'éviter de faire tourner l'aimant du distracteur à plaques lorsque cela n'est pas voulu, notamment lors de la phase de mise en place de l'outil d'activation en regard de l'aimant du distracteur à plaques ;

- le logement d'écrantage magnétique est en un alliage présentant une grande susceptibilité magnétique, par exemple en Permalloy®, ou en un matériau ferromagnétique, notamment en un matériau ferromagnétique doux ;
- le logement peut comporter un diaphragme en un matériau d'écrantage magnétique à son extrémité qui est fermé lorsque le logement est en configuration fermée et ouvert lorsque le logement es en configuration ouverte ;
- l'outil d'activation comporte un organe d'ouverture pour faire passer l'extrémité du logement d'écrantage magnétique de la configuration fermée à ouverte et inversement.

Il est également décrit un procédé de commande de l'allongement d'un distracteur à plaques tel que décrit ci-avant dans toutes ses combinaisons, au moyen d'un outil d'activation comprenant un aimant permanent, aimanté selon une direction transversale, comprenant les étapes consistant à :
i. placer l'aimant permanent de l'outil d'activation en regard de l'aimant permanent du distracteur, de telle sorte que leurs directions d'aimantation soient sensiblement parallèles ; et
ii. faire pivoter sur lui-même l'aimant permanent de l'outil d'activation, autour d'un axe sensiblement colinéaire à l'axe de l'aimant permanent du distracteur.

L'outil d'activation peut être tel que décrit précédemment.

Dans l'étape i., l'extrémité du logement d'écrantage magnétique est, de préférence, en configuration fermée, le procédé comportant une étape i'. consistant à faire passer l'extrémité du logement d'écrantage magnétique de la configuration fermée à la configuration ouverte.

Selon un dernier aspect, il est proposé un procédé de distraction osseuse comprenant les étapes de :
- fracturer l'os à allonger ;
- fixer sur l'os fracturé, les plaques de fixation d'un distracteur à plaques tel que décrit précédemment dans toutes ses combinaisons, les plaques de fixation étant fixées de part et d'autre de la fracture de l'os ; et
- commander l'allongement du distracteur à plaques en mettant en œuvre le procédé de commande de l'allongement d'un distracteur un plaques tel que décrit ci-avant.

La fracture de l'os peut être réalisée sous forme d'un trait d'ostéotomie, les plaques de fixation étant alors fixées de part et d'autre du trait d'ostéotomie.

D'autres caractéristiques et avantages de l'invention apparaîtront à la lecture de la description détaillée qui suit, laquelle description fait référence aux dessins ci-annexés parmi lesquels :
- La figure 1 représente schématiquement en coupe un premier exemple de distracteur à plaques, dans une configuration repliée ;
- La figure 2 illustre la commande de l'allongement du distracteur à plaques de la figure 1 au moyen d'un outil d'activation, vus en coupe ;
- La figure 3 est une figure analogue à la figure 1, dans laquelle le distracteur est dans une configuration allongée ;
- La figure 4 illustre schématiquement un deuxième exemple de distracteur à plaques ;
- la figure 5 représente schématiquement un troisième exemple de distracteur à plaques.
- la figure 6 illustre schématiquement en coupe une variante de distracteur à plaques, dans une configuration partiellement repliée ;
- la figure 7 illustre schématiquement le distracteur à plaques de la figure 6 vu du côté des plaques ;
- la figure 8 représente schématiquement en coupe une variante de distracteur à plaques ;
- la figure 9 est une représentation schématique d'un outil d'activation comportant un logement d'écrantage magnétique en configuration fermée ;
- la figure 10 représente l'outil d'activation de la figure 9, le logement étant en configuration ouverte,
- la figure 11 représente schématiquement en coupe une variante de distracteur à plaque, et
- la figure 12 représente schématiquement et en coupe transversale une variante d'aimant permanent dans son logement.

Dans la suite de la description, les éléments identiques ou de fonction identique portent le même signe de référence. À fin de concision de la présente description, ces éléments identiques ne sont pas décrits en détails, en regard de chaque figure, seules les différences entre les différents exemples étant décrites.

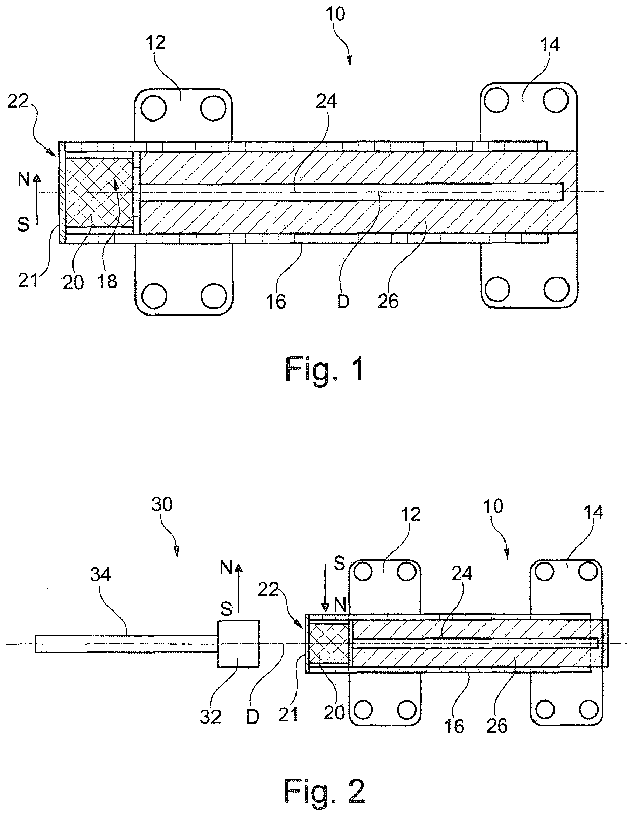

La figure 1 illustre un premier exemple de distracteur maxillo-facial à plaques 10.

Ce distracteur 10 comporte tout d'abord une première 12 et une deuxième 14 plaques de fixation sur un os à traiter. Ces plaques 12, 14 sont destinées à être fixées sur l'os traité, de part et d'autre de la fracture. Ces plaques 12, 14 sont sensiblement planes. Elles peuvent être chantournées pour s'adapter à l'os sur lequel elles sont à fixer. Ces plaques peuvent être d'épaisseur inférieure ou égale à 1,6 mm, mieux à 1,2 mm, de préférence à 1 mm et de préférence encore inférieure ou égale à 0,6 mm. Ces plaques 12, 14 peuvent par exemple être vissées sur l'os à traiter. Les vis mis en œuvre dans ce cas peuvent notamment être en titane, en alliage de titane ou en matériau résorbable. On préfère généralement utiliser des vis en alliage de titane en combinaison avec des vis résorbables. La longueur des vis peut par exemple être supérieure à 3 mm et/ou inférieure à 10 mm. Par exemple, les vis sont longues de 5 mm. Ici, les plaques 12, 14 présentent quatre trous pour positionner autant de vis. Bien entendu, ce nombre de trous n'est nullement limitatif. Par exemple, une plaque peut être fixée au moyen d'une vis en alliage de titane et d'une vis en matériau plastique résorbable. Les dimensions des plaques sont réduites, notamment pour une application maxillo-faciale, puisque l'espace disponible est réduit, pour limiter la gêne occasionnée par le distracteur à plaques. Les plaques présentent par exemples une longueur et une largeur comprises, chacune, entre 5 et 30 mm.

La première plaque 12 est fixée à une douille 16, par exemple par soudage ou collage. En variante, la première plaque 12 et la douille 16 sont monobloc. Ici, la douille 16 présente à une extrémité un logement creux 18 recevant un aimant permanent 20. L'aimant 20 est reçu libre en rotation autour d'un axe D. L'aimant 20 est fixe en translation par rapport à la douille 16 et donc la première plaque 12.

De préférence, le logement 18 est hermétiquement clos, pour éviter que l'aimant 20 ne puisse entrer en contact avec le milieu environnant. Ici, ceci est réalisé en fixant une plaque 21 fermant le logement 18 après l'insertion de l'aimant 20. La plaque 21 peut notamment être soudée ou collée. La fermeture du logement est particulièrement intéressante dans le cas où l'aimant 20, par exemple en néodyme ou en alliage de néodyme, en samarium ou en alliage de samarium, en ferrite ou en alliage de ferrite, n'est pas biocompatible. En variante, l'aimant 20 peut être enrobé d'un matériau biocompatible, notamment du titane ou de la silicone. Dans ce cas, le logement peut être ouvert.

Ici, l'aimant permanent est cylindrique d'axe D. Plus précisément, l'aimant 20 est à section à symétrie de révolution, notamment à section circulaire. L'aimant 20 est aimanté selon une direction transverse à l'axe D. En d'autres termes, l'orientation Nord-Sud de l'aimant permanent correspond à une direction perpendiculaire à son axe D. L'aimant 20 est ici adapté à pivoter autour de son axe D.

Il peut être noté ici que, l'aimant permanent 20 peut, plus généralement, s'étendre selon une direction principale et être aimanté selon une direction transverse par rapport à la direction principale. L'aimant 20 peut ainsi notamment être cylindrique, à symétrie de révolution ou non, ou sphérique.

Il est à noter également ici que l'aimant 20 est disposé à proximité d'une extrémité de la douille 16 et, par la même, du distracteur à plaques 10. L'aimant permanent est en effet disposé de préférence à proximité d'une extrémité 22 du distracteur à plaques 10, notamment à une distance, mesurée selon la direction de l'axe D de rotation de l'aimant 20, inférieure ou égale à 15 mm, de préférence inférieure ou égale à 10 mm, de préférence encore inférieure ou égale à 5 mm. Selon un mode de réalisation non représenté, l'aimant 20 est même situé à l'extrémité du distracteur à plaques. Dans ce cas, l'extrémité du distracteur à plaques est formée par un côté de l'aimant 20.

L'aimant 20 est ici solidaire d'une tige filetée 24. Par exemple, la tige filetée est collée sur l'aimant 20. En variante, l'aimant 20 et la tige filetée 24 sont monobloc. La tige filetée 24 s'étend sensiblement selon l'axe D de rotation de l'aimant permanent. La tige filetée 24 est par ailleurs reçue dans un tube taraudé 26. Le tube taraudé 26 est solidaire de la deuxième plaque 14. Par exemple, le tube taraudé 26 est collé ou soudé sur la deuxième plaque 14. En variante, la deuxième plaque 14 et le tube taraudé 26 sont monoblocs. La douille 16 est creuse, de section complémentaire à la section du tube taraudé 26, de sorte que le tube taraudé 26 est partiellement reçu dans la douille 16. Le tube taraudé 26 est monté coulissant dans la douille 16.

Il est à noter que selon l'exemple de la figure 1, l'axe de rotation de l'aimant 20 et l'axe de la tige 24 sont confondus. Cette solution est compacte et est donc particulièrement adaptée à une application maxillo-faciale, notamment à une application mandibulaire. Cette solution est en outre simple techniquement. Ceci réduit les coûts, d'une part, et les risques de défaillance, d'autre part.

À fin de biocompatibilité et de légèreté du distracteur à plaques, au moins l'un parmi la tige filetée 24, le tube taraudé 26, la première plaque 12, la deuxième plaque 14 et la douille 16 est en titane ou en alliage de titane. De préférence, tous ces éléments sont en titane ou en alliage de titane.

Un tel distracteur à plaque peut être mis en œuvre de manière classique dans un procédé de distraction osseuse, notamment de distraction d'un os maxillo-facial, plus particulièrement encore de la mandibule.

Dans un premier temps, un praticien réalise un trait d'ostéotomie d'un os à allonger. Il s'agit en fait ici de réaliser une fracture chirurgicale de l'os à allonger.

Le praticien fixe ensuite les première 12 et deuxième 14 plaques du distracteur à plaques 10 sur l'os à allonger, de part et d'autre du trait d'ostéotomie.

Ensuite, le praticien ou tout utilisateur commande l'allongement du distracteurs à plaques 10. Pour ce faire, il peut avoir recours à un outil d'activation 30, comprenant essentiellement un aimant permanent 32 fixé sur une tige 34. De même que l'aimant 20, l'aimant 32 s'étend de préférence selon une direction principale et est aimanté selon une direction d'aimantation transverse par rapport à la direction principale. L'aimant 32 peut être cylindrique, de préférence à section transversale à symétrie de révolution, ou sphérique. Ici, l'aimant 32 est cylindrique à section transversale circulaire. Par suite, l'aimant 32 étant aimanté selon une direction d'aimantation transversale, l'orientation Nord-Sud est perpendiculaire à son axe principal.

La commande de l'allongement du distracteur à plaques 10 est alors réalisée comme suit. Dans un premier temps, on place l'aimant 32 en regard de l'aimant 20. Cette étape peut être facilitée par exemple par un marquage à l'encre sur la peau du patient, lors de la mise en place du distracteur. La mise en place des aimants 20, 32 en regard implique que leurs axes principaux, c'est-à-dire leurs axes de rotation, soient confondus, comme illustré à la figure 2. Il est à noter ici que du fait de l'orientation de la magnétisation des deux aimants 20, 32, il peut apparaître un faible effort de traction ou de repoussement de l'un vers l'autre. Cependant cet effort est faible comparé au cas où les aimants interagissent selon la direction de leurs axes d'aimantation. En outre, dans le cas de l'exemple décrit en regard des figures 1 à 3, cet effort est sensiblement parallèle aux plaques de fixation du distracteur. Dans ces directions parallèles aux plaques, les vis de fixation des plaques 12, 14 ont une résistance aux contraintes importantes, empêchant ainsi un arrachement du distracteur. Au contraire, dans le cas où ces efforts sont dans la direction perpendiculaire aux plaques comme dans le cas des distracteurs connus, alors ils provoquent une force de traction sur les vis de fixation des plaques 12, 14. Ceci est susceptible de provoquer une douleur au patient, voire l'arrachement des plaques 12, 14.

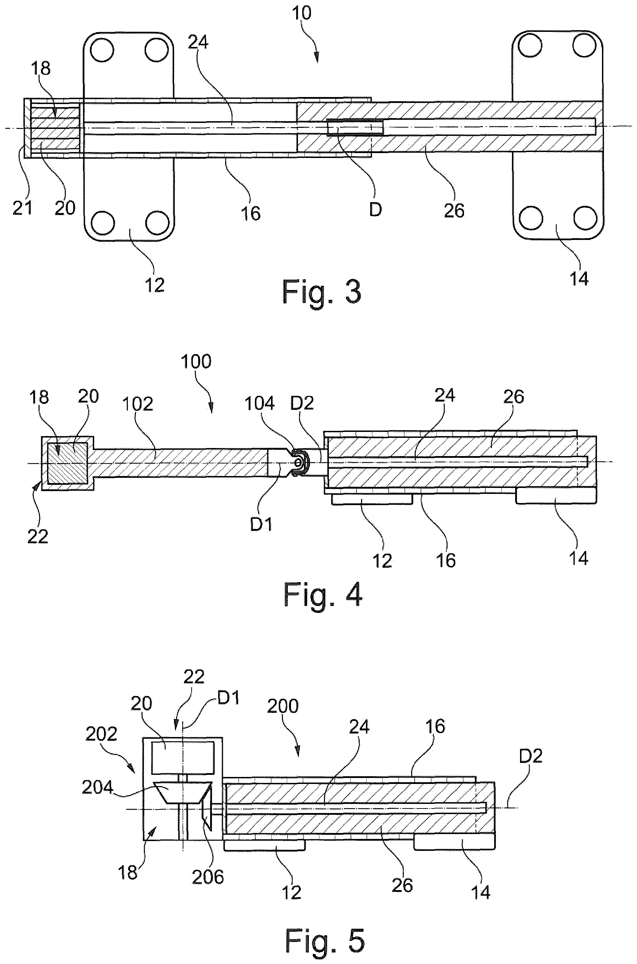

Le procédé d'allongement du distracteur se poursuit alors en faisant tourner l'outil d'activation 30 sur lui-même, autour de l'axe de l'aimant 32. On provoque ainsi une rotation de l'aimant 20 dans le distracteur à plaques 10. La tige filetée 24 étant fixée sur l'aimant 20, elle est également entrainée en rotation. Par contre, le tube taraudé 26 est fixé sur l'os à traiter. Il ne peut donc pas pivoter. Par suite, la rotation de la tige filetée 24 dans le tube taraudé 26 provoque un mouvement de translation du tube taraudé 26 par rapport à la douille 16. On allonge ainsi le distracteur à plaques 10, comme illustré à la figure 3. Ce faisant, on accroît la distance entre les deux plaques 12, 14.

Il est à noter ici que l'outil de commande 30 est particulièrement simple. Du fait de la commande magnétique de l'allongement du distracteur à plaques 10, il est possible de commander cet allongement depuis l'extérieur d'un patient, notamment depuis l'extérieur de la bouche d'un patient. La gêne occasionnée est donc fortement réduite. En outre, la commande est réalisée par une rotation de cet outil sur lui-même, autour de l'axe de l'aimant 32 cylindrique. Cette commande peut donc être réalisée même dans des espaces restreints ou difficiles d'accès. Il n'est pas nécessaire d'avoir recours à un outil de commande devant être disposé tout autour de l'os à traiter. Il n'y a pas non plus de variation de couple sur une rotation d'un tour de l'aimant 32 permettant une commande uniforme, sans à-coups.

Le distracteur à plaques 100 de la figure 4 se distingue du distracteur à plaques 10 des figures 1 à 3 en ce que le logement 18 recevant l'aimant 20 est formé dans un bras 102, l'aimant 20 étant solidaire en rotation avec le bras 102. En outre, le bras 102 est relié à la tige filetée 24 par une liaison rotule à doigt. Pour ce faire, un joint de cardan 104 est ici prévu entre le bras 102 et la tige filetée 24. Ceci offre la possibilité de décaler angulairement l'axe de rotation D1 de l'aimant 20 et l'axe D2 de la tige filetée 24. Ceci peut être particulièrement utile dans le cas d'un os à traiter difficile d'accès. Il est à noter que même dans ce cas, l'aimant 20 reste situé à proximité de l'extrémité 22 du distracteur à plaques 100, cette extrémité 22 étant ici formée par le bras 102.

Le fonctionnement de ce distracteur à plaque 100 est sensiblement identique à celui du distracteur à plaques 10. La rotation de l'aimant 20, commandé comme cela a été décrit précédemment, provoque la rotation du bras 102. La rotation du bras 102 est transmise via le joint de cardan 104 à la tige filetée 24. Comme le tube taraudé 26, solidaire de la deuxième plaque 14, ne peut pas tourner en même temps que la tige filetée 24, cette rotation de la tige filetée 24 provoque un mouvement de sortie du tube taraudé 26 hors de la douille 16, augmentant ainsi la distance entre les deux plaques 12, 14.

Le distracteur à plaques 200 de la figure 5 se distingue du distracteur à plaques 10 des figures 1 à 3 en ce qu'il comporte un dispositif de renvoi d'angle 202. Ici, en effet, l'aimant 20 tourne autour d'un axe de rotation D1 qui est sensiblement perpendiculaire à l'axe D2 de la tige 24. Pour ce faire, l'aimant 20 est monté solidaire en rotation avec une première roue dentée conique 104, laquelle engrène une deuxième roue dentée conique 106, solidaire en rotation de la tige 24. Bien sûr, d'autres types de roues dentées peuvent être mises en œuvre dans ce cas.

Il est à noter ici qu'il est possible, en choisissant le nombre de dents des première et deuxième roues dentées 204, 206 d'obtenir un effet de réduction. Notamment il est possible d'obtenir une réduction de la vitesse de rotation de la tige 24 par rapport à l'aimant 20, qui s'accompagne d'une augmentation du couple transmis à la tige 24. Ceci peut être intéressant pour favoriser l'allongement du distracteur à plaques 200.

On peut noter ici, que même dans le cas du distracteur à plaques 200, l'aimant permanent est disposé à proximité d'une extrémité 22 du distracteur à plaques 200. En effet, ici aussi, l'aimant permanent est disposé à une distance de l'extrémité 22 du distracteur à plaques 200, mesurée selon la direction de l'axe de rotation D1 de l'aimant, inférieure ou égale à 15 mm, de préférence inférieure ou égale à 10 mm, de manière plus préférée inférieure ou égale à 5 mm. L'aimant 20 peut même être au niveau de l'extrémité 22 du distracteur à plaques 200, notamment dans le cas où l'aimant est recouvert d'un matériau biocompatible.

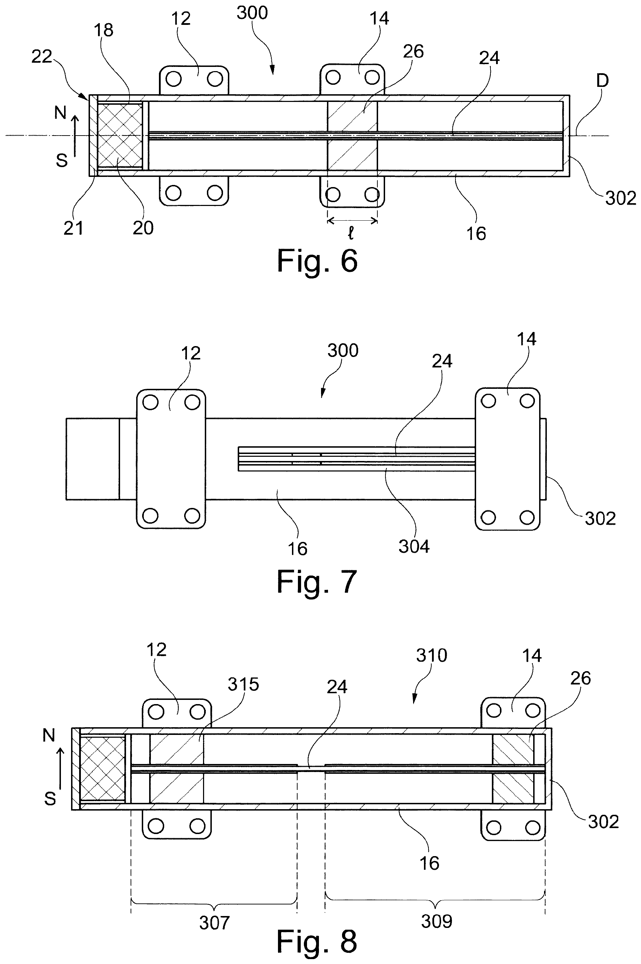

Le distracteur à plaques 300 des figures 6 et 7 diffère du distracteur à plaques 10 des figures 1 à 3 en ce que le tube taraudé 26 est de faible encombrement et la douille 16 est fermée à son extrémité 302 opposée à celle de l'aimant 20. Le tube taraudé 26 est, de préférence, d'une largeur *l* inférieure à celle de la plaque sur laquelle il est monté. Lors de la rotation de la tige filetée 24, le tube taraudé 26 se déplace en translation le long de la tige filetée en coulissant à l'intérieur de la douille 16 qui sert donc de guide. La douille 16 comporte une ouverture longitudinale 304, s'étendant sur au moins une partie de la longueur du distracteur à plaques, et dans laquelle l'ensemble plaque 14/tube taraudé 26 coulisse. Un tel tube taraudé 26 de taille réduite permet de réduire les frottements entre le tube, la douille 16 et la tige 24 lors du déplacement du tube taraudé 26.

Le distracteur à plaques 310 de la figure 8 diffère de celui des figures 6 et 7 en ce qu'un deuxième tube taraudé 315 est monté sur la première plaque 12 et en ce que la tige filetée présente deux zones longitudinales 317 et 319 présentant des orientations de filetage inversées. Ainsi, lors de de la rotation de la tige filetée, les deux tubes 26 et 315 se déplacent dans des directions opposées de sorte à écarter ou rapprocher, selon le sens de rotation, les plaques 12 et 14.

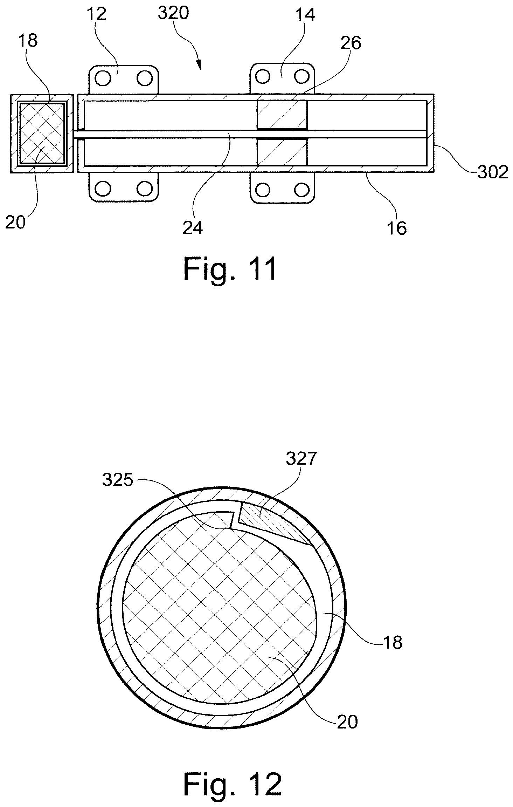

Le distracteur à plaques 310 de la figure 11 diffère de celui des figures 6 et 7 en ce que le logement 18 est formé par un boitier 322 hermétiquement clos relié à la tige filetée 24 et dissocié de la douille 16. Lors de la rotation de l'aimant 20, le boitier 322 tourne avec ce dernier au moins dans un sens et entraîne ainsi la tige filetée 24 en rotation. Un tel boîtier permet notamment de protéger l'aimant de l'environnement extérieur.

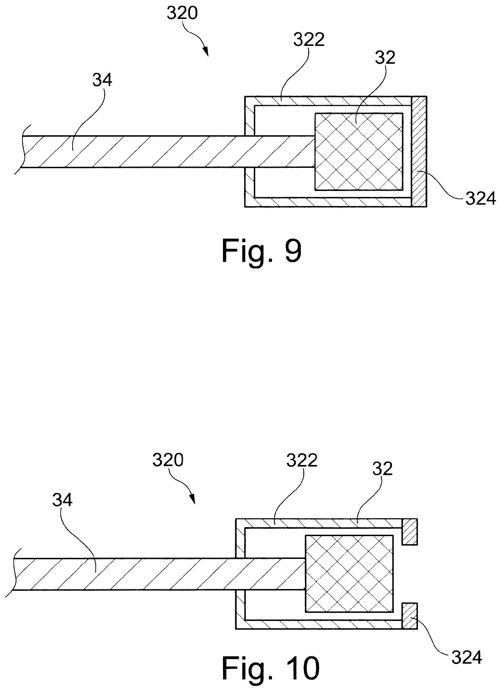

Comme cela est illustré sur les figures 9 et 10, l'outil d'activation 320 peut présenter un boîtier 322 en un matériau d'écrantage magnétique, notamment en Permalloy® ou en un matériau paramagnétique doux présentant à son extrémité 324 un diaphragme pouvant passer d'une configuration fermée empêchant le passage du champ magnétique de l'aimant 32 à une configuration ouverte permettant au moins partiellement le passage du champ magnétique de l'aimant 32.

Ainsi, lors de l'utilisation de l'outil d'activation, l'utilisateur peut positionner l'outil d'activation en configuration fermée en regard de l'aimant 20 du distracteur ouvrir le diaphragme lorsque l'outil d'activation est bien positionné. Ceci évite que l'aimant 32 de l'outil n'agisse sur l'aimant 20 sans que cela ne soit désiré.

Le passage de la configuration fermée à la configuration ouverte peut s'effectuer à l'aide d'un bouton non représenté, présent sur la tige 34 de l'outil.

Bien entendu, l'invention n'est pas limitée aux exemples décrits ci-avant, mais est susceptible de nombreuses variantes accessibles à l'homme de l'art, dans le cadre de la définition donnée par les revendications ci-jointes.

Ainsi, l'aimant permanent 20 peut être solidaire en rotation du tube taraudé et la tige filetée montée fixe par rapport à la douille 16.

La deuxième plaque 14 peut être plus proche de l'aimant permanent que la deuxième plaque 12.

Le distracteur à plaques peut comprendre un dispositif anti-retour empêchant la rotation de l'aimant dans un sens provoquant un raccourcissement de la distance entre les première et deuxième plaques de fixation. Le dispositif anti-retour peut notamment être du type à cliquet. Ceci présente en effet l'avantage supplémentaire de pouvoir commander précisément la mesure de l'allongement de la distance entre les première et deuxième plaques.

En variante illustré sur la figure 12, l'aimant permanent 20 comporte à sa surface une encoche asymétrique 325 et le logement 18 comporte une lame 327, l'encoche 325 et la lame 327 étant configurées pour que lorsque l'aimant 20 est entraîné en rotation dans le sens antihoraire, l'aimant est libre en rotation dans le logement et n'entraîne donc pas en rotation la tige filetée et lorsque l'aimant 20 est entraîné en rotation dans le sens horaire, la lame 327 vient en prise avec l'encoche 325 et le logement 18 est entraîné en rotation avec l'aimant 20, ce qui fait entraîne en rotation la tige filetée 24.

En variante encore, le système anti-retour peut être adapté sur l'outil d'activation de sorte que ce dernier ne puisse tourner que dans un sens.

En outre, le distracteur à plaques peut comporter un dispositif de réduction, notamment pour accroître le couple entre la tige et le tube taraudé. Ce dispositif de réduction peut notamment comprendre un ou plusieurs engrenages de roues dentées. Il est à noter ici que, dans le cas où un dispositif de réduction est prévu, il est préféré pour des raisons d'encombrement, que l'axe de rotation de l'aimant permanent reste sensiblement parallèle à l'axe de la tige filetée.

En outre, le distracteur à plaques selon l'invention peut comporter un dispositif de détermination de la longueur d'allongement du distracteur. Ce dispositif permet ainsi de vérifier à chaque commande de l'allongement, que cette commande est conforme à la valeur souhaitée de l'allongement. Il peut ainsi être prévu un dispositif à cliquet, tel que la rotation de la tige filetée par rapport au tube provoque l'émission de clics. Le compte du nombre de clics permet de déterminer l'allongement commandé. Le dispositif de détermination de la longueur d'allongement peut être indépendant du dispositif anti-retour. En variante, cependant, un seul dispositif à cliquet peut permettre à la fois de déterminer la longueur d'allongement du distracteur et empêcher la rotation de la tige filetée dans un sens commandant le rétrécissement de la distance entre les plaques du distracteur.

De manière plus générale, le dispositif de détermination de la longueur d'allongement peut être conformé pour émettre un son à chaque tour ou portion de tour, notamment à chaque demi-tour ou à chaque quart de tour.

Enfin, le distracteur à plaques peut comporter plus de deux plaques. Notamment, il peut comporter trois plaques, par exemple dans le cas où il est mis en œuvre pour une distraction dite de « transport d'os ». Dans ce cas, deux des plaques sont fixées de part et d'autre du trait d'ostéotomie, comme décrit précédemment. La troisième plaque est alors fixée au tube taraudé, d'une part, et à l'os, d'autre part, de chaque côté du trait d'ostéotomie.

## Revendications

1. Ensemble de distraction comprenant :
- un distracteur à plaques (10 ; 100 ; 200), notamment distracteur maxillo-facial à plaques, comportant :
o une première (12) et une deuxième (14) plaques de fixation,
o une tige filetée (24),
o un tube taraudé (26) monté sur la tige filetée et fixé sur la deuxième plaque de fixation (12),
o une douille (16) recevant la tige filetée (24) libre en rotation et fixe en translation et le tube taraudé (26) fixe en rotation et libre en translation,
o un aimant permanent (20) reçu dans un logement (18), s'étendant selon une direction principale et aimanté selon une direction transversale à la direction principale, monté dans le distracteur à plaques (10 ; 100 ; 200) de telle sorte que la rotation de l'aimant permanent (20) sur lui-même, autour d'un axe de rotation (D ; D1) parallèle à l'axe principal, provoque une rotation relative de la tige filetée (24) par rapport au tube taraudé (26), l'aimant permanent (20) étant situé à une distance d'une des extrémités (22) du distracteur à plaques (10 ; 100 ; 200), mesurée selon la direction de l'axe de rotation (D ; D1) de l'aimant permanent (20), inférieure ou égale à 15 mm, de préférence l'aimant permanent (20) du distracteur étant situé à une extrémité (22) du distracteur à plaques (10),
- un outil (30) d'activation du distracteur à plaques comprenant un aimant permanent (32), s'étendant selon une direction principale (D ; D1) et aimanté selon une direction transversale à la direction principale,
**caractérisé en ce que** l'aimant permanent (32) de l'outil d'activation (30) est configuré pour que, lorsqu'il est placé en regard de l'aimant permanent (20) du distracteur, les directions d'aimantation (NS ; SN) de l'aimant permanent (32) de l'outil d'activation (30) et de l'aimant permanent (20) du distracteur soient sensiblement parallèles et l'aimant permanent (32) de l'outil d'activation (30) permettant d'entraîner l'aimant permanent (20) du distracteur en rotation en faisant pivoter sur lui-même l'aimant permanent (32) de l'outil d'activation autour d'un axe (D ;D1) sensiblement colinéaire à l'axe de l'aimant permanent (20) du distracteur.

2. Ensemble selon la revendication 1, dans lequel l'aimant (20) du distracteur est cylindrique, de préférence à symétrie de révolution, ou sphérique.

3. Ensemble selon l'une des revendications 1 à 2, le distracteur comprenant un dispositif de renvoi d'angle (202) de telle sorte que l'axe de rotation (D1) de l'aimant permanent (20) soit sensiblement perpendiculaire à l'axe (D2) de la tige filetée (24) et du tube taraudé (26), le dispositif de renvoi d'angle (202) comportant de préférence une première roue dentée (204), solidaire en rotation de l'aimant permanent (20) et adaptée à engrener une deuxième roue dentée (206) commandant la rotation de l'un parmi la tige filetée (24) et le tube taraudé (26) et/ou dans lequel l'aimant permanent (20) du distracteur est relié à la tige filetée (24) ou au tube taraudé (26) par un dispositif de réduction, notamment à engrenage.

4. Ensemble selon la revendication 3, dans lequel le dispositif de réduction inclut, de préférence est constitué par, les première (204) et deuxième (206) roues dentées.

5. Ensemble selon l'une quelconque des revendications précédentes, le distracteur comprenant un bras (102) relié au tube fileté (26) ou, de préférence, à la tige filetée (24), par une liaison rotule à doigt et le dispositif de réduction de couple, le cas échéant, l'aimant permanent (20) étant fixé sur le bras (102), la liaison rotule a doigt étant de préférence réalisée au moyen d'un joint de cardan (104).

6. Ensemble selon l'une des revendications 1 à 3, dans lequel l'aimant permanent (20) du distracteur est solidaire en rotation de la tige filetée (24) ou du tube taraudé (26), l'aimant permanent (20) étant de préférence fixé à la tige filetée (24) ou au tube taraudé (26).

7. Ensemble selon l'une quelconque des revendications précédentes, le distracteur comprenant un dispositif anti-retour empêchant la rotation de l'aimant permanent (20) du distracteur dans un sens commandant une réduction de la distance entre les première (12) et deuxième (14) plaques, le dispositif anti-retour étant de préférence du type à cliquet.

8. Ensemble selon l'une quelconque des revendications précédentes, dans lequel l'aimant permanent (20) du distracteur est en néodyme ou en un alliage de néodyme, en samarium ou alliage de samarium, en ferrite ou alliage de ferrite, de préférence enrobé d'un matériau biocompatible, et/ou dans lequel au moins l'un parmi, de préférence chacun parmi la tige filetée (24), le tube taraudé (26), la première plaque (12) et la deuxième plaque (14) est en titane ou en alliage de titane.

9. Ensemble selon l'une quelconque des revendications précédentes, dans lequel le logement (18) est fixé à la tige filetée (24) et/ou le logement (18) est hermétiquement clos.

10. Ensemble selon l'une quelconque des revendications précédentes, dans lequel le tube taraudé (26) présente une largeur inférieure ou égale à la largeur de la deuxième plaque (14).

11. Ensemble selon l'une quelconque des revendications précédentes, dans lequel l'aimant permanent (20) du distracteur est tel que son aimantation est conservée lorsqu'il est chauffé à une température de 120°C, mieux de 130°C, encore mieux de 134°C.

12. Ensemble selon l'une quelconque des revendications précédentes, dans lequel la tige filetée (24) est montée libre en rotation et solidaire en translation par rapport à la première plaque (12) de fixation ou dans lequel la tige filetée (24) comporte deux zones longitudinales (307, 309) ayant des filetages inversés, le premier tube taraudé (26) sur lequel est fixé la deuxième plaque (14) étant monté sur une des deux zones longitudinales (309) et un deuxième tube taraudé (315) sur lequel est fixé la première plaque (12) étant monté sur l'autre des deux zones longitudinales (307).

13. Ensemble selon l'une quelconque des revendications précédentes, dans lequel l'aimant permanent (20) du distracteur et le logement (18) sont configurés pour que l'aimant permanent soit libre en rotation dans le logement lorsqu'il tourne dans un premier sens et que l'aimant permanent entraîne en rotation le logement et de ce fait la tige filetée lorsqu'il tourne en rotation dans un second sens, notamment l'aimant permanent comportant une encoche asymétrique et le logement comportant un relief saillant coopérant avec l'encoche de sorte que l'aimant permanent soit libre de tourner dans le premier sens et que le relief vienne en prise avec l'encoche dans le second sens.

14. Ensemble selon l'une quelconque des revendications précédentes, le distracteur comprenant en outre un dispositif de détermination de la longueur d'allongement du distracteur, notamment à cliquet.

15. Ensemble selon la revendication 1, dans lequel l'outil d'activation comporte un logement d'écrantage magnétique (322) dans lequel l'aimant permanent (32) de l'outil d'activation est reçu, le logement d'écrantage magnétique (322) pouvant passer d'une configuration fermée dans laquelle l'écrantage du champ magnétique est suffisante pour que la rotation de l'aimant permanent (32) de l'outil d'activation autour de la direction principale n'entraîne pas de rotation de l'aimant permanent (20) du distracteur à plaques autour de son axe à une configuration ouverte dans laquelle la rotation de l'aimant permanent (32) de l'outil d'activation autour de la direction principale entraîne la rotation de l'aimant permanent (20) du distracteur à plaques autour de son axe et inversement ou dans lequel l'aimant permanent (32) de l'outil d'activation (30) est cylindrique, de préférence à symétrie de révolution, ou sphérique.

## Patentansprüche

1. Distraktionsanordnung, umfassend:
- einen Plattendistraktor (10; 100; 200), insbesondere Maxillo-Fazial-Plattendistraktor, mit:
o einer ersten Befestigungsplatte (12) und einer zweiten Befestigungsplatte (14),
o einer Gewindestange (24),
o einem mit Innengewinde versehenen Rohr (26), das auf der Gewindestange montiert und auf der zweiten Befestigungsplatte (14) befestigt ist,
o einer Buchse (16), welche die Gewindestange (24) frei drehbar und translationsfest und das mit Innengewinde versehene Rohr (26) drehfest und translatorisch frei aufnimmt,
o einem Dauermagneten (20), der in einer Aufnahme (18) aufgenommen ist, sich in einer Hauptrichtung erstreckt und in einer Richtung quer zu der Hauptrichtung magnetisiert ist, der in dem Plattendistraktor (10; 100; 200) so montiert ist, dass die Drehung des Dauermagneten (20) um sich selbst, um eine Drehachse (D; D1) parallel zu der Hauptachse, eine relative Drehung der Gewindestange (24) gegenüber dem mit Innengewinde versehenen Rohr (26) bewirkt, wobei der Dauermagnet (20) sich in einem Abstand von einem der Enden (22) des Plattendistraktors (10; 100; 200) befindet, der, gemessen in der Richtung der Drehachse (D; D1) des Dauermagneten (20), kleiner oder gleich 15 mm ist, wobei der Dauermagnet (20) des Distraktors sich vorzugsweise an einem Ende (22) des Plattendistraktors (10) befindet,
- ein Werkzeug (30) zur Aktivierung des Plattendistraktors, umfassend einen Dauermagneten (32), der sich in einer Hauptrichtung (D; D1) erstreckt und in einer Richtung quer zu der Hauptrichtung magnetisiert ist,
**dadurch gekennzeichnet, dass** der Dauermagnet (32) des Aktivierungswerkzeugs (30) eingerichtet ist, damit, wenn er dem Dauermagneten (20) des Distraktors gegenüber platziert ist, die Magnetisierungsrichtungen (NS; SN) des Dauermagneten (32) des Aktivierungswerkzeugs (30) und des Dauermagneten (20) des Distraktors im Wesentlichen parallel sind und wobei der Dauermagnet (32) des Aktivierungswerkzeugs (30) gestattet, den Dauermagneten (20) des Distraktors zur Drehung anzutreiben, indem er bewirkt, dass der Dauermagnet (32) des Aktivierungswerkzeugs sich um sich selbst um eine Achse (D; D1) dreht, die zu der Achse des Dauermagneten (20) des Distraktors im Wesentlichen kollinear ist.

2. Anordnung nach Anspruch 1, wobei der Dauermagnet (20) des Distraktors zylindrisch, vorzugsweise rotationssymmetrisch, oder kugelförmig ist.

3. Anordnung nach einem der Ansprüche 1 bis 2, wobei der Distraktor eine Eckumleitungsvorrichtung (202) umfasst, in der Form, dass die Drehachse (D1) des Dauermagneten (20) zu der Achse (D2) der Gewindestange (24) und des mit Innengewinde versehenen Rohrs (26) im Wesentlichen senkrecht ist, wobei die Eckumleitungsvorrichtung (202) vorzugsweise ein erstes Zahnrad (204) aufweist, das mit dem Dauermagneten (20) drehfest verbunden ist und eingerichtet ist, um mit einem zweiten Zahnrad (206) in Eingriff zu kommen, das die Drehung einer/eines von Folgenden: der Gewindestange (24) und dem mit Innengewinde versehenen Rohr (26), steuert, und/oder wobei der Dauermagnet (20) des Distraktors mit der Gewindestange (24) oder mit dem mit Innengewinde versehenen Rohr (26) durch eine Reduzierungsvorrichtung, insbesondere mit Getriebe, verbunden ist.

4. Vorrichtung nach Anspruch 3, wobei die Reduzierungsvorrichtung das erste Zahnrad (204) und das zweite Zahnrad (206) umfasst, vorzugsweise aus ihnen besteht.

5. Anordnung nach einem der vorhergehenden Ansprüche, der Distraktor einen Arm (102) umfasst, der mit dem mit Innengewinde versehenen Rohr (26), oder, vorzugsweise, mit der Gewindestange (24), durch eine Fingerkugelgelenkverbindung und gegebenenfalls die Drehmomentreduzierungsvorrichtung verbunden ist, wobei der Dauermagnet (20) auf dem Arm (102) befestigt ist, wobei die Fingerkugelgelenkverbindung vorzugsweise mittels eines Kardangelenks (104) realisiert ist.

6. Anordnung nach einem der Ansprüche 1 bis 3, wobei der Dauermagnet (20) des Distraktors mit der Gewindestange (24) oder dem mit Innengewinde versehenen Rohr (26) drehfest verbunden ist, wobei der Dauermagnet (20) vorzugsweise an der Gewindestange (24) oder an dem mit Innengewinde versehenen Rohr (26) befestigt ist.

7. Anordnung nach einem der vorhergehenden Ansprüche, der Distraktor eine Rücklaufsperre umfasst, welche die Drehung des Dauermagneten (20) des Distraktors in einer Richtung verhindert, die eine Reduzierung des Abstands zwischen der ersten Platte (12) und der zweiten Platte (14) steuert, wobei die Rücklaufsperre vorzugsweise des Sperrklinkentyps ist.

8. Anordnung nach einem der vorhergehenden Ansprüche, wobei der Dauermagnet (20) des Distraktors aus Neodym oder aus einer Neodym-Legierung, aus Samarium oder Samarium-Legierung, aus Ferrit oder Ferrit-Legierung ist, vorzugsweise mit einem biokompatiblen Material umhüllt, und/oder wobei wenigstens eine/eines, vorzugsweise jede/jedes von Folgenden: der Gewindestange (24), dem mit Innengewinde versehenen Rohr (26), der ersten Platte (12) und der zweiten Platte (14), aus Titan oder aus Titan-Legierung ist.

9. Anordnung nach einem der vorhergehenden Ansprüche, wobei die Aufnahme (18) an der Gewindestange (24) befestigt ist und/oder die Aufnahme (18) hermetisch verschlossen ist.

10. Anordnung nach einem der vorhergehenden Ansprüche, wobei das mit Innengewinde versehene Rohr (26) eine Breite aufweist, die kleiner oder gleich der Breite der zweiten Platte (14) ist.

11. Anordnung nach einem der vorhergehenden Ansprüche, wobei der Dauermagnet (20) des Distraktors dergestalt ist, dass seine Magnetisierung bewahrt wird, wenn er auf eine Temperatur von 120 °C, besser von 130 °C, noch besser von 134 °C erhitzt wird.

12. Anordnung nach einem der vorhergehenden Ansprüche, wobei die Gewindestange (24) gegenüber der ersten Befestigungsplatte (12) frei drehbar und translationsfest ist, oder wobei die Gewindestange (24) zwei längsgerichtete Zonen (307, 309) mit gegenläufigen Gewinden aufweist, wobei das erste mit Innengewinde versehene Rohr (26), auf dem die zweite Platte (14) befestigt ist, auf einer der zwei längsgerichteten Zonen (309) montiert ist, wobei ein zweites mit Innengewinde versehenes Rohr (315), auf dem die erste Platte (12) befestigt ist, auf der anderen der zwei längsgerichteten Zonen (307) montiert ist.

13. Anordnung nach einem der vorhergehenden Ansprüche, wobei der Dauermagnet (20) des Distraktors und die Aufnahme (18) eingerichtet sind, damit der Dauermagnet sich in der Aufnahme frei drehen kann, wenn er sich in einer ersten Richtung dreht, und dass der Dauermagnet die Aufnahme und dadurch die Gewindestange zur Drehung antreibt, wenn er sich in einer zweiten Richtung dreht, wobei der Dauermagnet insbesondere eine asymmetrische Einkerbung aufweist, und wobei die Aufnahme eine Erhöhung aufweist, die mit der Einkerbung so zusammenwirkt, dass der Dauermagnet sich in ersten Richtung frei drehen kann und dass die Erhöhung in der zweiten Richtung mit der Einkerbung in Eingriff kommt.

14. Anordnung nach einem der vorhergehenden Ansprüche, wobei der Distraktor ferner eine Vorrichtung zur Bestimmung der Dehnungslänge des Distraktors aufweist, insbesondere mit Sperrklinke.

15. Anordnung nach Anspruch 1, wobei das Aktivierungswerkzeug eine magnetische Abschirmungsaufnahme (322) aufweist, in welcher der Dauermagnet (32) des Aktivierungswerkzeugs aufgenommen ist, wobei die magnetische Abschirmungsaufnahme (322) übergehen kann von einer geschlossenen Stellung, in der die Abschirmung des Magnetfelds ausreichend ist, damit die Drehung des Dauermagneten (32) des Aktivierungswerkzeugs um die Hauptrichtung nicht die Drehung des Dauermagneten (20) des Plattendistraktors um seine Achse bewirkt, in eine geöffnete Stellung, in der die Drehung des Dauermagneten (32) des Aktivierungswerkzeugs um die Hauptrichtung die Drehung des Dauermagneten (20) des Plattendistraktors um seine Achse bewirkt und umgekehrt, oder wobei der Dauermagnet (32) des Aktivierungswerkzeugs (30) zylindrisch, vorzugsweise rotationssymmetrisch, oder kugelförmig ist.

## Claims

1. Distraction assembly comprising:
- a plate distractor (10; 100; 200), in particular a maxillofacial plate distractor, comprising:
• first (12) and second (14) attachment plates,
• a threaded rod (24),
• a tapped tube (26) mounted on the threaded rod and attached to the second attachment plate (12),
• a bush (16) receiving the threaded rod (24) free in rotation and fixed in translation and the tapped tube (26) fixed in rotation and free in translation,
• a permanent magnet (20) received in a housing (18), extending along a main direction and magnetised along a direction transverse to the main direction, mounted in the plate distractor (10; 100; 200) such that rotation of the permanent magnet (20) about itself, about an axis of rotation (D; D1) parallel to the main axis, causes a relative rotation of the threaded rod (24) relative to the tapped tube (26), the permanent magnet (20) being located at a distance from one of the ends (22) of the plate distractor (10; 100; 200), measured along the direction of the axis of rotation (D; D1) of the permanent magnet (20), less than or equal to 15 mm, preferably the permanent magnet (20) of the distractor being located at an end (22) of the plate distractor (10),
- a tool (30) for activating the plate distractor comprising a permanent magnet (32), extending along a main direction (D; D1) and magnetised along a direction transverse to the main direction,
**characterised in that** the permanent magnet (32) of the activation tool (30) is configured so that, when it is placed opposite the permanent magnet (20) of the distractor, the magnetisation directions (NS; SN) of the permanent magnet (32) of the activation tool (30) and of the permanent magnet (20) of the distractor are substantially parallel and the permanent magnet (32) of the activation tool (30) being able to drive the permanent magnet (20) of the distractor in rotation by pivoting about itself the permanent magnet (32) of the activation tool about an axis (D; D1) substantially colinear with the axis of the permanent magnet (20) of the distractor.

2. Assembly according to claim 1, wherein the magnet (20) of the distractor is cylindrical, preferably with symmetry of revolution, or spherical.

3. Assembly according to one of claims 1 to 2, the distractor comprising an angle gear box device (202) such that the axis of rotation (D1) of the permanent magnet (20) is substantially perpendicular to the axis (D2) of the threaded rod (24) and the tapped tube (26), the angle gear box device (202) preferably comprising a first gear (204) integral in rotation with the permanent magnet (20) and adapted to mesh with a second gear (206) controlling the rotation of one from the threaded rod (24) and the tapped tube (26) and/or wherein the permanent magnet (20) of the distractor is connected to the threaded rod (24) or to the tapped tube (26) by a reduction device, in particular geared.

4. Assembly according to claim 3, wherein the reduction device includes, preferably consists of, the first (204) and second (206) gears.

5. Assembly according to any one of the preceding claims, the distractor comprising an arm (102) connected to the tapped tube (26) or, preferably, to the threaded rod (24), by a finger ball joint and the torque reduction device, if any, the permanent magnet (20) being attached to the arm (102), the finger ball joint preferably consisting of a universal joint (104).

6. Assembly according to one of claims 1 to 3, wherein the permanent magnet (20) of the distractor is integral in rotation with the threaded rod (24) or the tapped tube (26), the permanent magnet (20) preferably being attached to the threaded rod (24) or the tapped tube (26).

7. Assembly according to any one of the preceding claims, the distractor comprising a non-return device preventing the rotation of the permanent magnet (20) of the distractor in a direction controlling a reduction of the distance between the first (12) and second (14) plates, the non-return device preferably being of the ratchet type.

8. Assembly according to any one of the preceding claims, wherein the permanent magnet (20) of the distractor is made of neodymium or a neodymium alloy, samarium or a samarium alloy, ferrite or ferrite alloy, preferably coated with a biocompatible material, and/or wherein at least one from, preferably each from the threaded rod (24), the tapped tube (26), the first plate (12) and the second plate (14) is made of titanium or a titanium alloy.

9. Assembly according to any one of the preceding claims, wherein the housing (18) is attached to the threaded rod (24) and/or the housing (18) is hermetically sealed.

10. Assembly according to any one of the preceding claims, wherein the width of the tapped tube (26) is less than or equal to the width of the second plate (14).

11. Assembly according to any one of the preceding claims, wherein the permanent magnet (20) of the distractor is such that its magnetisation is preserved when it is heated to a temperature of 120 °C, preferably 130 °C, more preferably 134 °C.

12. Assembly according to any one of the preceding claims, wherein the threaded rod (24) is mounted free in rotation and integral in translation relative to the first attachment plate (12) or wherein the threaded rod (24) comprises two longitudinal areas (307, 309) having reverse threads, the first tapped tube (26) to which the second plate (14) is attached being mounted on one of the two longitudinal areas (309) and a second tapped tube (315) to which the first plate (12) is attached being mounted on the other of the two longitudinal areas (307).

13. Assembly according to any one of the preceding claims, wherein the permanent magnet (20) of the distractor and the housing (18) are configured so that the permanent magnet is free to rotate in the housing when it turns in a first direction and so that the permanent magnet drives in rotation the housing and therefore the threaded rod when it rotates in a second direction, in particular the permanent magnet comprising an asymmetrical notch and the housing comprising a projecting relief cooperating with the notch such that the permanent magnet is free to rotate in the first direction and such that the relief engages with the notch in the second direction.

14. Assembly according to any one of the preceding claims, the distractor further comprising a device for determining the length of elongation of the distractor, in particular of the ratchet type.

15. Assembly according to claim 1, wherein the activation tool comprises a magnetic shielding housing (322) in which the permanent magnet (32) of the activation tool is received, the magnetic shielding housing (322) being able to switch from a closed configuration in which the magnetic field shielding is sufficient to prevent the rotation of the permanent magnet (32) of the activation tool about the main direction from driving in rotation the permanent magnet (20) of the plate distractor about its axis to an open configuration in which the rotation of the permanent magnet (32) of the activation tool about the main direction drives the rotation of the permanent magnet (20) of the plate distractor about its axis and vice versa or in which the permanent magnet (32) of the activation tool (30) is cylindrical, preferably with symmetry of revolution, or spherical.
